# EUROPÄISCHE PATENTSCHRIFT

(11) **EP 1 539 264 B1**
(45) Veröffentlichungstag und Bekanntmachung des Hinweises auf die Patenterteilung: **23.12.2009**
(21) Anmeldenummer: 03766332.5
(22) Anmeldetag: 28.07.2003
(51) Int. Cl.: A61L 27/38, C12M 3/06

(54) **VERFAHREN UND VORRICHTUNG ZUM ZÜCHTEN VON ZELLEN**
METHOD AND DEVICE FOR CULTURING CELLS
PROCEDE ET DISPOSITIF DE CULTURE DE CELLULES

(30) Priorität: 30.07.2002 DE 10234742
(43) Veröffentlichungstag der Anmeldung: 15.06.2005
(73) Patentinhaber: Bader, Augustinus, 04668 Parthenstein / OT Klinga (DE)
(72) Erfinder: Bader, Augustinus, 04668 Parthenstein / OT Klinga (DE)
(74) Vertreter: Benz, Jürgen
(86) Internationale Anmeldenummer: PCT/EP2003/008325
(87) Internationale Veröffentlichungsnummer: WO 2004/012782

(56) Entgegenhaltungen:
- WO-A-02/24861
- DE-A- 10 104 008
- DE-A- 19 719 751
- DE-A- 19 935 643

## Beschreibung

Die Erfindung betrifft ein Verfahren zum Züchten von Zellen, wobei die Zellen zur Bildung einer Zellschicht in einen Zellkulturraum eingeleitet werden.

In der DE 199 35 643 A1 ist ein Verfahren und eine Vorrichtung zum Züchten von Zellen beschrieben, wobei Zellen auf einem Träger in einem formbaren Zellkulturraum zwischen Folien gezüchtet werden. Der Träger ist dabei mit den Folien in einem Behälter als Bioreaktor eingebracht, wobei Nährstoffe und Sauerstoff von außen zugeführt werden.

Ein ähnliches Verfahren und eine Vorrichtung hierzu ist auch aus der DE 197 19 751 A1 bekannt. Das Aufwachsen der Zellen erfolgt dabei mehr oder weniger "unkontrolliert". Die Größe der Vorrichtung wird durch den Behälter vorgegeben, in welchem sich die zu bildende Zellschicht bzw. ein Implantat befindet.

Der vorliegenden Erfindung liegt die Aufgabe zugrunde, ein Verfahren zum Züchten von Zellen zu schaffen, das sehr vielseitig einsetzbar ist, insbesondere wobei aus den Zellen gebildete Formen, wie Implantate oder Prothesen, in sehr komplexer Form und in exakt definierbarer Größe hergestellt werden können.

Erfindungsgemäß wird dies bei einem Verfahren zum Züchten von Zellen, wobei die Zellen zur Bildung einer Zellschicht in einen Zellkulturraum eingeleitet werden, derart gelöst, dass der Zellkulturraum im Inneren einer Trägerstruktur gebildet wird, wobei die Trägerstruktur in ihrer Form und Größe wenigstens annähernd der durch die Zellen zu bildenden Form, wie einem Implantat oder einer Prothese, entspricht, wobei der Trägerstruktur Nährstoffe und/oder Sauerstoff zugeführt wird und wobei die Trägerstruktur außenseitig mit einer gegenüber Zellen undurchlässigen Grenzschicht versehen wird.

Erfindungsgemäß stellt nunmehr die Trägerstruktur den eigentlichen Bioreaktor selbst dar, während bisherige Bioreaktoren lediglich bestimmte einfache geometrische Formen, wie z.B. rund oder quadratisch, flach oder flaschenförmig, besaßen. Durch die außenseitig gegenüber den Zellen undurchlässige Grenzschicht wird eine exakt definierte Zellkulturraumgröße und Form gegeben, wobei die Form und Größe selbst durch die Trägerstruktur vorgegeben wird. Dies bedeutet, man kann die Trägerstruktur exakt in der Form ausbilden, wie die zu bildende Form, z.B. das Implantat oder die Prothese, später in ihrem Endzustand aussehen soll. Das herzustellende Implantat ist praktisch vorgegeben. So kann man z.B. mit einem Computertomogramm, mit welchem z.B. ein defekter Wirbelkörper erkannt wird, diesen exakt als Trägerstruktur nachbilden. In die Trägerstruktur, die entsprechend mit der Grenzschicht versehen worden ist, werden dann die Zellen entsprechend eingebracht. Vorzugsweise ist hierzu die Trägerstruktur aus einem mikroporösen oder auch aus einem grobporösen Material gebildet. Dabei kann die Trägerstruktur als entfernbares oder auch als umwandelbares Platzhaltermaterial ausgebildet sein, so dass sich die Zellschicht entsprechend dem gewünschten Implantat ausbilden kann.

Die Grenzschicht kann aus einem gegenüber den Zellen undurchlässigen Kunststoff gebildet sein, und hierfür z.B. durch Aufspritzen oder durch Tauchbad aufgebracht werden. Als geeignet hat sich z.B. flüssiges oder viskoses Polymer, Silikone, Polyurethane, Proteine, Alginate oder Harze herausgestellt.

Alternativ kann die Grenzschicht auch aus einem biologischen Material gebildet werden, wie z.B. aus einem Hydrogel oder Alginat. Bei Verwendung eines Alginates kann dieses in einer Kalziumchloridlösung polymerisiert und damit gegenüber Zellen undurchlässig gemacht werden. Zur nachfolgenden Entfernung und nach Fertigstellung des Implantates kann das polymerisierte Alginat in eine kalziumarme Lösung eingebracht werden, womit es sich wieder auflöst.

Sofern keine selbstauflösende Grenzschicht verwendet wird, kann man diese auch auf mechanische Weise nach Ende des Zellenzüchtverfahrenes entfernen.

Vorteilhafte Weiterbildungen und Ausgestaltungen der Erfindung ergeben sich aus den übrigen Unteransprüchen und aus den nachfolgend anhand der Zeichnung beschriebenen Ausführungsbeispielen.

Es zeigt:
- Fig. 1: einen Wirbelkörper als zu bildendes Implantat in stark vereinfachter Darstellung;
- Fig. 2: ein Tauchbad für den Wirbelkörper nach der Fig. 1;
- Fig. 3: den Wirbelkörper nach der Fig. 1 mit einer Grenz- schicht;
- Fig. 4: ein Lösungsbad für die Grenzschicht;
- Fig. 5: einen Behälter mit einer Nährmittellösung mit mehre- ren Implantaten;
- Fig. 6: ein Implantat in Röhrenknochenform;
- Fig. 7: ein Teilimplantat für eine Herzklappe;
- Fig. 8: das in der Fig. 3 dargestellte Implantat in einem Nährmittelkreislauf;
- Fig. 9: das in der Fig. 3 dargestellte Implantat in einem unter Druck setzbaren Behälter;
- Fig. 10: eine Trägerstruktur in Form eines Kniegelenkes in einem Behälter; und
- Fig. 11: die Trägerstruktur nach der Fig. 10 mit einer zu- sätzlichen Meniskusstruktur.

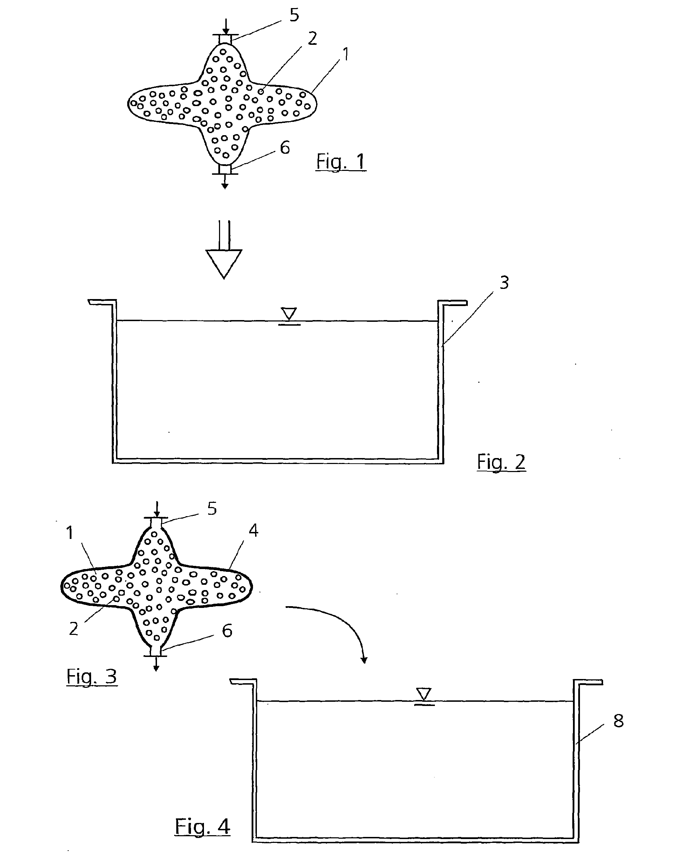

In der Fig. 1 ist schematisch die Form eines Wirbelkörpers dargestellt, an Hand dem nachfolgend die Erfindung näher beschrieben wird. Selbstverständlich ist der Wirbelkörper nur als ein mögliches Beispiel für eine Prothese oder ein Implantat anzusehen. Ausgangspunkt für den Wirbelkörper stellt eine Trägerstruktur 1 dar, die aus einem porösen Material, z.B. mikroporös oder auch grobporös, besteht. Als Material für die Trägerstruktur 1 kann ein stabiles, biodegradables oder auch remodelling-fähiges Material verwendet werden. So kann z.B. Knochenersatzmaterial oder auch Kalziumphosphat verwendet werden. Auch Kunststoffe und hybride Konstrukte, wobei ein technisches Material mit einem biologischen Material kombiniert wird, sind möglich. Wesentlich ist lediglich, dass Materialien verwendet werden, die gegenüber einzubringenden Zellen 2 inert sind bzw. die eingebrachten Zellen nicht schädigen oder die sich von den Zellen 2 "umbauen" lassen.

Damit ein definierter Zellkulturraum im Inneren der Trägerstruktur 1 für die Zellen 2 gegeben ist und damit die Größe und Form des zu bildenden Implantates eingehalten wird, ist dafür zu sorgen, dass die Außenwand der Trägerstruktur 1 gegenüber Zellen undurchlässig ist. Hierzu kann die Trägerstruktur 1 vor dem Einbringen der Zellen 2 z.B. in ein Tauchbad 3 (siehe Fig. 2) eingetaucht werden. Das Tauchbad 3 kann z.B. ein flüssiges oder viskoses Polymer sein, das eine Grenzschicht 4 (siehe Fig. 3) für die ansonsten poröse Trägerstruktur 1 bildet. Als Polymere zur Bildung einer Grenzschicht können z.B. Harze verwendet werden.

Anstelle einem Polymer kann zur Verkapselung der Trägerstruktur 1 durch Bildung einer Grenzschicht 4 auch ein Alginatmaterial verwendet werden, das in einer Kalziumchloridlösung in dem Tauchbad 3 polymerisiert wird. Eine derartige Grenzschicht 4 ist biologisch verträglich.

Die Grenzschicht 4 kann absolut dicht ausgebildet sein. Von Vorteil ist es jedoch, wenn sie wenigstens gaspermeabel ausgebildet ist. In diesem Falle kann durch die Grenzschicht 4 hindurch Sauerstoff eingebracht werden. Ebenso ist es auch möglich, eine Grenzschicht 4 zu bilden, die derart mikroporös ist, dass auch Nährstoffe durch die Grenzschicht 4 hindurch in das Innere der Trägerstruktur 1 diffundieren bzw. ein Stoffaustausch mit der Trägerstruktur, d.h. dem späteren Implantat, erfolgt.

Zur Zufuhr von Zellen 2, Nährmedium und gegebenenfalls einem Sauerstoffträger-Medium, z.B. Fluoridlösungen, Blut oder Blutersatzstoffe, kann die Trägerstruktur 1 mit einem Zulaufanschluss 5 versehen sein. Falls über den Zufuhranschluss 5 auch Nährstoffe in die Trägerstruktur 1 eingebracht werden sollen, kann man die Trägerstruktur 1 auch mit einem Ablaufanschluss 6 versehen, womit eine Durchströmung gegeben ist. Den Zulaufanschluss 5 und den Ablaufanschluss 6 kann man an die Trägerstruktur 1 vor dem Tauchbad 3 oder auch nach dem Tauchbad 3 einbringen, wobei selbstverständlich dafür zu sorgen ist, dass in dem Zulauf- und dem Auslaufbereich keine undurchlässige Grenzschicht 4 vorgesehen wird.

Die Trägerstruktur 1 kann als entfernbares oder auch als umwandelbares Platzhaltermaterial für die einzubringenden Zellen 2 ausgebildet sein. So kann z.B. für die Trägerstruktur 1 ein Material verwendet werden, das bei Zugabe von körpereigenen Zellen aufgelöst wird, z.B. durch Enzyme. Auf diese Weise wird eine körpereigene Matrix gebildet, die patientenspezifisch ist.

Als Grenzschicht 4 kann auch ein Gel verwendet werden, das eine geschlossene Membran als Grenzschicht bildet.

Nach Beendigung des Zellbildungsprozesses bzw. des aus den Zellen 2 gebildeten Implantates muss die Grenzschicht 4 wieder entfernt werden. Wenn sie aus biologischem Material ist, kann man sie entsprechend wieder biologisch abbauen, wie dies z.B. bei Gelen oder Alginaten der Fall ist. Hierzu kann die Trägerstruktur 1 mit der Grenzschicht 4 gemäß Fig. 4 wiederum in ein Bad 7 eingetaucht werden, das die Grenzschicht 4 abbaut. Bei Verwendung von Alginaten kann man hierfür eine Lösung verwenden, die Kalzium entzieht, so dass sich die Grenzschicht 4 entsprechend auflöst. Die Grenzschicht 4 kann auch so ausgebildet sein, dass sie sich durch enzymatische oder hydrolytische Prozesse selbst auflöst. Die Grenzschicht 4 kann auch vaskularisiert oder vorvaskularisiert (z.B. durch Endoter- oder Stammzellen) sein.

Bei Verwendung von Kunststoffen oder Silikon, welche sich nicht auf einfache Weise auflösen lassen, kann man die Grenzschicht 4 gegebenenfalls auch auf mechanische Weise entfernen. Um diese Entfernung zu erleichtern, kann zwischen der Trägerstruktur 1 und der Grenzschicht 4 eine Zwischenschicht angeordnet werden, die keine Bindung mit dem Material der Trägerstruktur 1 eingeht. Durch diese Zwischenschicht lässt sich dann die Grenzschicht 4 leichter ablösen. Hierfür kann z.B. eine Lipidschicht, eine Protein- und/oder Albuminschicht oder andere auflösbare oder ablösbare Schichten (biodegradabel oder erosionsfähige Schichten) verwendet werden.

Statt einer Zuführung von Zellen 2 über den Zulaufanschluss 5 kann gegebenenfalls auch die Trägerstruktur 1 z.B. in eine wässrige oder pastöse Lösung eingetaucht werden, in der.sich Zellen 2 zusammen mit Nährlösung befinden. In diesem Falle saugt sich die Trägerstruktur 1 entsprechend mit Zellen 2 und Nährlösung voll. Anschließend erfolgt die Verkapselung durch eine Grenzschicht 4 in dem Tauchbad 3.

Anstelle eines Tauchbades 3 kann selbstverständlich auch eine Grenzschicht 4 als Barriere für die Zellen 2 aufgespritzt oder aufgestrichen werden.

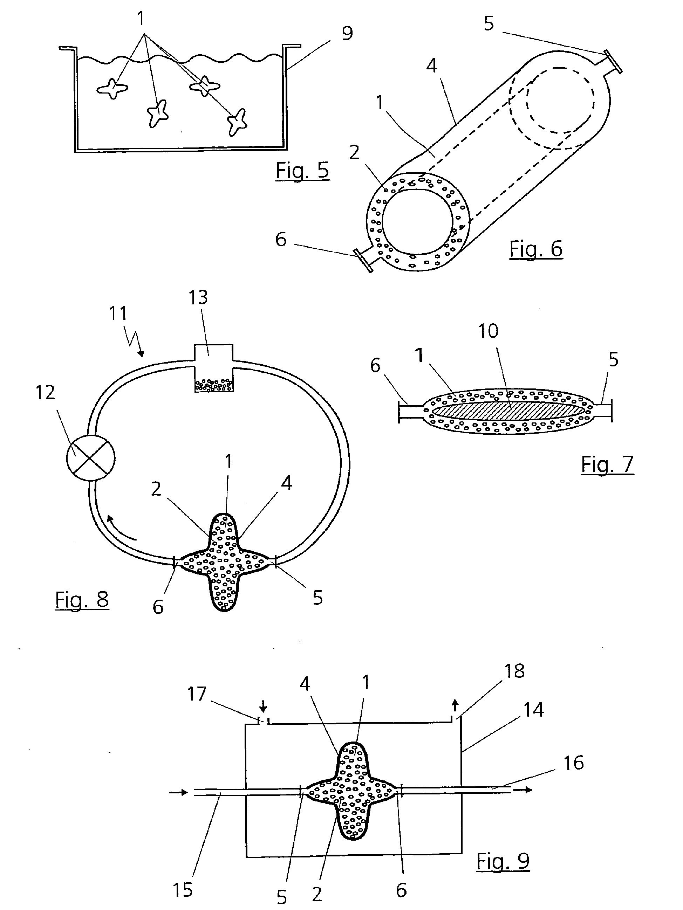

In Fig. 5 ist eine Ausgestaltung vorgesehen, wobei mehrere mit Grenzschichten 4 versehene Trägerstrukturen 1 in ein Nähermittelbad 9 eingebracht werden, damit der Wachstumsprozess in Gang gerät. Gegebenenfalls kann auch hier zusätzlich für eine Sauerstoffzufuhr in das Nährmittelbad 9 gesorgt werden.

In der Fig. 6 ist als Anwendungsbeispiel eine Trägerstruktur 1 in Form eines Röhrenknochens dargestellt, der ebenfalls innen- und außenseitig mit der Grenzschicht 4 versehen ist und ebenfalls einen Zulaufanschluss 5 und einen Ablaufanschluss 6 besitzen kann. In das Innere der Trägerstruktur 1 kann man dann als Zellen Stammzellen einbringen, die aus dem Knochenmark stammen, welche man z.B. mit Biopsie gewinnen kann. Die Stammzellen bilden dann aus dem fremden Trägerstrukturmaterial zunehmend zeitabhängig ein eigenes Knochenmaterial. Selbstverständlich muss das Material der Trägerstruktur 1 dann entsprechend auflösbar, z.B. aus Kalziumphosphat, gebildet sein.

Die Fig. 7 zeigt ausschnittsweise eine Herzklappe mit einem Edelstahlteil, z.B. Titanteil 10, um das außen die Trägerstruktur 1 angeordnet ist, wobei ebenfalls ein Zulaufanschluss 5 und ein Ablaufanschluss 6 vorgesehen sein können. In diesem Falle wird zusammen mit dem Titanteil 10 eine Trägerstruktur 1 vorgegeben, die der Form einer Herzklappe nachgebildet ist. Anstelle der Verwendung von Titan 10 kann auch eine Polyurethanprothese als Herzklappe verwendet werden.

Die Fig. 8 zeigt prinzipmäßig die Anordnung einer Trägerstruktur 1 mit Zellen 2 und einer Grenzschicht 4 in einem Kreislauf 11 mit einer Pumpe 12 und einem Medienreservoir 13. In dem Medienreservoir 13 können Zellen und/oder Nährlösung angeordnet sein. In den Kreislauf 11 kann auch ein Sauerstoffträger eingebunden werden.

Die Fig. 9 zeigt die Anordnung einer Trägerstruktur 1 in einem Behälter 14, in den eine Zulaufleitung 15 für Nährstoffe und/oder Sauerstoff mündet, welche mit dem Zulaufanschluss 5 verbunden ist. Eine Ablaufleitung 16 führt aus dem Behälter 14 heraus und ist mit dem Ablaufanschluss 6 verbunden.

Zusätzlich ist der Behälter 14 mit einem Anschluss 17 zur Einleitung von Druckmittel und gegebenenfalls auch mit einem Auslauf 18 zu dessen Ableitung versehen. Als Druckmittel kann ein Gas oder ein flüssiges Medium verwendet werden.

Es hat sich nämlich herausgestellt, dass die Bildung einer Zellschicht und das Zellwachstum deutlich verbessert wird, wenn man die Zellen 2 einer Druckbelastung aussetzt. Auf diese Weise werden noch bessere in-vivo-Verhältnisse geschaffen.

Durch die Druckbeaufschlagung der Trägerstruktur 1 über den unter Druck gesetzten Behälter 14 wird eine großflächige Druckbeaufschlagung erreicht, welche in-vivo-Verhältnisse sehr gut simuliert. Dabei können die Druckbelastungen auch wechselnd aufgebracht werden.

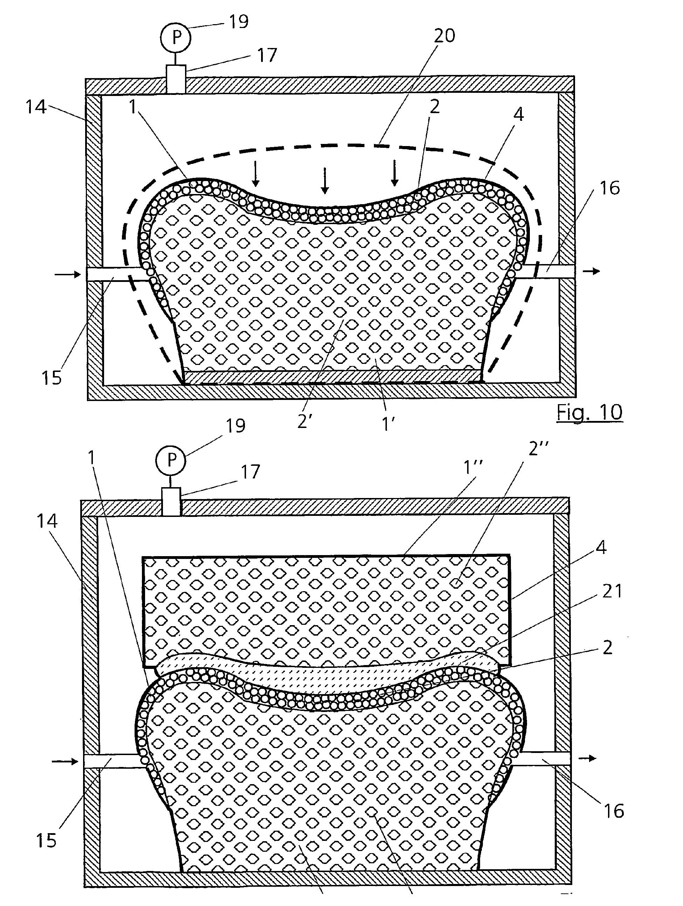

Die Fig. 10 zeigt hierfür eine mögliche Ausführungsform für Gelenkknorpeln, die auf einer Trägerstruktur 1, welche ein Kniegelenk darstellen soll, gezüchtet werden.

Für das Kniegelenk, welches ebenfalls eine Trägerstruktur 1' z.B. aus Trikalziumphosphat sein kann, können ebenfalls Zellen 2' auf nicht näher dargestellte Weise eingebracht werden. Gegebenenfalls kann zur Abgrenzung auch zwischen der Trägerstruktur 1' und der Trägerstruktur 1, in welcher Knorpelzellen 2 gezüchtet werden, eine Grenzschicht eingebracht werden, damit eine klar Trennung zwischen den Zellen 2 und 2' hergestellt wird.

Über den Zulaufanschluss 17 erfolgt eine Druckbeaufschlagung des Inneren des Behälters 14 durch eine Pumpe 19. Durch die Pumpe 19 können wechselnde Drücke in den Innenraum des Behälters 14 eingebracht werden. Wie dargestellt, ist in diesem Falle ein Ablaufanschluss 18 nicht unbedingt vorhanden.

Wie gestrichelt in der Fig. 10 angedeutet, kann um die beiden Trägerstrukturen 1 und 1' zusätzlich noch eine Schutzfolie 20 angeordnet sein. Die Schutzfolie 20 kann für einen Transport der Einheit aus den beiden Trägerstrukturen 1 und 1' vorgesehen sein und diese Einheit entsprechend steril abschließen. Durch die Ausbildung als elastisch dehnbare Folie 20 ist sichergestellt, dass die durch die Pumpe 19 aufgebrachte Druckbelastung auf die Trägerstrukturen 1 und 1' weitergeleitet wird.

Anstelle von Trikalziumphosphat für die Trägerstrukturen 1 und 1' können im Knochenersatzbereich auch Collagene verwendet werden, wobei z.B. auch ein Meniskus gezüchtet werden kann. Ebenso sind Bindegewebsstrukturen, Polymere, wie Polylaktide oder andere chemische Strukturen, verwendbar. Wesentlich ist lediglich, dass sich aus diesen Materialien Formen erstellen lassen, die dem gewünschten Implantat entsprechen.

Zusätzlich lässt sich der Behälter 14 im Bedarfsfalle auch mit elektrischen Anschlüssen versehen, durch die man über nicht dargestellte Verbindungsleitungen elektrische Impulse den Zellen 2 und 2' auferlegen kann, womit ebenfalls noch besser in-vivo-Simulationen erzielt werden können.

Die in der Fig. 11 dargestellte Ausführungsform entspricht im wesentlichen der in Fig. 10 besprochenen Form, weshalb auch für die gleichen Teile die gleichen Bezugszeichen beibehalten worden sind.

Unterschiedlich ist lediglich, dass zusätzlich noch eine Meniskusstruktur 21 aufgebracht worden ist, über die wiederum eine Trägerstruktur 1" liegt.

Zur Vereinfachung ist in diesem Falle eine gegen Zellen 2 undurchlässige Grenzschicht 4 über die gesamte Einheit geschaffen.

Die Grenzschicht 4 kann auch durch Zellen selbst gebildet werden, die man dazu benutzt, ein Membran zu züchten. Auf der Trägerstruktur 1 wird dann z.B. Bindegewebe als Umkapselung gebildet. Dies kann z.B. durch Überwachsungsprozesse mit Zellen, wie z.B. Chondrozyten, Fibroblasten oder Osteoblasten, erfolgen. Diese Zellen stellen dann praktisch Verpackungszellen und eine Grenzschicht für die zu züchtenden Zellen 2 dar.

## Patentansprüche

1. Verfahren zur Herstellung von aus Zellen gebildeten Implantaten von definierbarer Größe, umfassend die Schritte:
(i) Bereitstellung einer mikroporösen oder grobporösen Trägerstruktur (1), welche Form und Größe des aus den eingesetzten Zellen (2) zu bildenden Implantats sowie eine außenseitig gegenüber den Zellen undurchlässige Grenzschicht (4) aufweist und als entfernbares oder umwandelbares Platzhaltermaterial für die Zellen ausgebildet ist,
(ii) Zuführung von Zellen und Nährmedium in die als Bioreaktor dienende Trägerstruktur und Züchten der Zellen, so dass sich eine Zellschicht in der Form des Implantats unter Entfernung oder Umwandlung des Platzhaltermaterials ausbildet, und
(iii) Entfernung der Grenzschicht (4) nach Beendigung des Zellzüchtungsverfahrens und Erhalt des aus gezüchteten Zellen erhaltenen Implantats.

2. Verfahren nach Anspruch 1, **dadurch gekennzeichnet, dass** die Trägerstruktur bioabbaubar ist.

3. Verfahren nach Anspruch 2, **dadurch gekennzeichnet, dass** die Trägerstruktur ein Knochenersatzmaterial oder Kalziumphosphat ist.

4. Verfahren nach Anspruch 1, **dadurch gekennzeichnet, dass** die Grenzschicht (4) aus einem synthetischen oder biologischen Material gebildet ist.

5. Verfahren nach Anspruch 4, **dadurch gekennzeichnet, dass** die Grenzschicht (4) aus einem Hydrogel gebildet ist.

6. Verfahren nach Anspruch 4, **dadurch gekennzeichnet, dass** die Grenzschicht (4) aus einem Alginat gebildet ist, welches in einer Kalziumchloridlösung polymerisiert und nach Bildung besagter Zellschicht durch eine kalziumarme Lösung von der Trägerstruktur(1) abgelöst wird.

7. Verfahren nach Anspruch 4, **dadurch gekennzeichnet, dass** die Grenzschicht (4) eine Überwachsung mit Zellen aufweist, die eine die Trägerstruktur (1) umkapselnde Membran bilden.

8. Verfahren nach Anspruch 1, **dadurch gekennzeichnet, dass** die Grenzschicht (4) gaspermeable ausgebildet ist.

9. Verfahren nach Anspruch 1, **dadurch gekennzeichnet, dass** die Grenzschicht (4) durch Aufspritzen oder durch ein Tauchbad (3) aufgebracht wird.

10. Verfahren nach Anspruch 1, **dadurch gekennzeichnet, dass** die Grenzschicht (4) mechanisch abziehbar ausgebildet ist.

11. Verfahren nach Anspruch 1, **dadurch gekennzeichnet, dass** die Grenzschicht (4) ablösbar oder auflösbar ausgebildet ist.

12. Verfahren nach Anspruch 1, **dadurch gekennzeichnet, dass** zwischen der Trägerstruktur (1) und der Grenzschicht (4) eine Zwischenschicht eingebracht ist, die keine Verbindung mit der Trägerstruktur hat.

13. Verfahren nach Anspruch 1, **dadurch gekennzeichnet, dass** die Trägerstruktur (1) durch flüssiges oder gasförmiges Medium Druckbelastungen ausgesetzt wird.

14. Verfahren nach Anspruch 13, **dadurch gekennzeichnet, dass** die Trägerstruktur (1) in einen Behälter (14) eingesetzt ist, der durch ein Druckmedium (19) einem wechselnden Gas- oder Flüssigkeitsdruck ausgesetzt wird.

15. Verfahren nach Anspruch 13 oder 14, **dadurch gekennzeichnet, dass** um die Trägerstruktur (1) eine Schutzfolie (20) gelegt ist, die einen Druckraum um diese bildet und auf der von der Trägerstruktur (1) abgewandten Seite Druckbelastungen ausgesetzt wird.

16. Verfahren nach Anspruch 1, **dadurch gekennzeichnet, dass** die Grenzschicht (4) außenseitig auf die Trägerstruktur (1) aufgebracht wird, nachdem die Trägerstruktur mit Zellen und Nährmedium versehen wurde.

17. Verfahren nach Anspruch 1, **dadurch gekennzeichnet, dass** die Trägerstruktur (1) mit Zu- und Abläufen (5, 6) für Nährstoffe versehen ist.

18. Verfahren nach Anspruch 17, **dadurch gekennzeichnet, dass** die Trägerstruktur (1) in einen Nährmittelkreislauf (11) eingesetzt ist.

19. Verwendung einer mikroporösen oder grobporösen Trägerstruktur als Bioreaktor zur Herstellung von Implantaten von definierbarer Größe, wobei die Trägerstruktur (1) als entfernbares oder umwandelbares, bioabbaubares Platzhaltermaterial für Zellen (2) ausgebildet ist, die in die Trägerstruktur eingebracht werden, und Form und Größe des aus den eingesetzten Zellen (2) zu bildenden implantats besitzt und außenseitig eine gegenüber den Zellen undurchlässige Grenzschicht (4) aufweist, welche nach Beendigung des Zellzüchtungsverfahrens entfernt wird.

## Claims

1. Method for the production of implants of definable size formed from cells, comprising the steps of:
(i) provision of a microporous or coarsely porous support structure (1) which has the shape and size of the implant to be formed from the cells (2) employed and a boundary layer (4) which is impermeable to the cells on the outside, and has been formed as a removable or convertible placeholder material for the cells,
(ii) feeding of cells and nutrient medium into the support structure serving as bioreactor and cultivation of the cells, so that a cell layer in the form of the implant forms on removal or conversion of the placeholder material, and
(iii) removal of the boundary layer (4) after completion of the cell cultivation method, giving the implant obtained from cultivated cells.

2. Method according to Claim 1, **characterized in that** the support structure is biodegradable.

3. Method according to Claim 2, **characterized in that** the support structure is a bone replacement material or calcium phosphate.

4. Method according to Claim 1, **characterized in that** the boundary layer (4) has been formed from a synthetic or biological material.

5. Method according to Claim 4, **characterized in that** the boundary layer (4) has been formed from a hydrogel.

6. Method according to Claim 4, **characterized in that** the boundary layer (4) has been formed from an alginate, which is polymerized in a calcium chloride solution and, after formation of said cell layer, detached from the support structure (1) by a low-calcium solution.

7. Method according to Claim 4, **characterized in that** the boundary layer (4) has an overgrowth of cells which form a membrane encapsulating the support structure (1).

8. Method according to Claim 1, **characterized in that** the boundary layer (4) has been formed in gas-permeable form.

9. Method according to Claim 1, **characterized in that** the boundary layer (4) is applied by spraying or by means of a dip bath (3).

10. Method according to Claim 1, **characterized in that** the boundary layer (4) has been formed in mechanically removable form.

11. Method according to Claim 1, **characterized in that** the boundary layer (4) has been formed in detachable or dissolvable form.

12. Method according to Claim 1, **characterized in that** an interlayer which is not bonded to the support structure has been introduced between the support structure (1) and the boundary layer (4).

13. Method according to Claim 1, **characterized in that** the support structure (1) is subjected to pressure loads by means of liquid or gaseous medium.

14. Method according to Claim 13, **characterized in that** the support structure (1) has been inserted into a container (14) which is subjected to a varying gas or liquid pressure by means of a pressure medium (19).

15. Method according to Claim 13 or 14, **characterized in that** a protective film (20) has been placed around the support structure (1) and forms a pressure space around the latter and is subjected to pressure loads on the side facing away from the support structure (1).

16. Method according to Claim 1, **characterized in that** the boundary layer (4) is applied to the outside of the support structure (1) after the support structure has been provided with cells and nutrient medium.

17. Method according to Claim 1, **characterized in that** the support structure (1) has been provided with supply and discharge inlets and outlets (5, 6) for nutrients.

18. Method according to Claim 17, **characterized in that** the support structure (1) has been inserted into a nutrient circuit (11).

19. Use of a microporous or coarsely porous support structure as bioreactor for the production of implants of definable size, where the support structure (1) has been formed as removable or convertible, biodegradable placeholder material for cells (2) which are introduced into the support structure, and has the shape and size of the implant to be formed from the cells (2) employed and has on the outside a boundary layer (4) which is impermeable to the cells and which is removed after completion of the cell cultivation method.

## Revendications

1. Procédé pour la production d'implants de taille définissable formés à partir de cellules, comprenant les étapes de :
(i) fourniture d'une structure de support microporeuse ou grossièrement poreuse (1) qui a la forme et la dimension de l'implant qui doit être formé à partir des cellules (2) employées et d'une couche de frontière (4) qui est imperméable aux cellules sur l'extérieur, et qui a été formée en tant que matériau d'accueil de colonisation enlevable ou convertible pour les cellules,
(ii) apport de cellules et d'un milieu de nutriment dans la structure de support servant de bioréacteur et de mise en culture des cellules, de telle sorte qu'une couche de cellules sous la forme de l'implant se forme lors de l'enlèvement ou de la conversion du matériau d'accueil de colonisation, et
(iii) enlèvement de la couche de frontière (4) après achèvement du processus de mise en culture des cellules, produisant ainsi l'implant obtenu à partir des cellules cultivées.

2. Procédé selon la revendication 1, **caractérisé en ce que** la structure de support est biodégradable.

3. Procédé selon la revendication 2, **caractérisé en ce que** la structure de support est un matériau de remplacement d'os ou un phosphate de calcium.

4. Procédé selon la revendication 1, **caractérisé en ce que** la couche de frontière (4) a été formée à partir d'un matériau synthétique ou biologique.

5. Procédé selon la revendication 4, **caractérisé en ce que** la couche de frontière (4) a été formée à partir d'un hydrogel.

6. Procédé selon la revendication 4, **caractérisé en ce que** la couche de frontière (4) a été formée à partir d'un alginate, qui est polymérisé dans une solution de chlorure de calcium et, après formation de ladite couche de cellules, qui est détaché de la structure de support (1) par une solution à faible teneur en calcium.

7. Procédé selon la revendication 4, **caractérisé en ce que** la couche de frontière (4) présente une surcroissance de cellules qui forment une membrane encapsulant la structure de support (1).

8. Procédé selon la revendication 1, **caractérisé en ce que** la couche de frontière (4) a été formée sous une forme perméable aux gaz.

9. Procédé selon la revendication 1, **caractérisé en ce que** la couche de frontière (4) est appliquée par pulvérisation ou au moyen d'un bain d'immersion (3).

10. Procédé selon la revendication 1, **caractérisé en ce que** la couche de frontière (4) a été formée sous une forme enlevable mécaniquement.

11. Procédé selon la revendication 1, **caractérisé en ce que** la couche de frontière (4) a été formée sous une forme détachable ou dissolvable.

12. Procédé selon la revendication 1, **caractérisé en ce qu'**une intercouche qui n'est pas liée à la structure de support a été introduite entre la structure de support (1) et la couche de frontière (4).

13. Procédé selon la revendication 1, **caractérisé en ce que** la structure de support (1) est soumise à des charges de pression au moyen d'un milieu liquide ou gazeux.

14. Procédé selon la revendication 13, **caractérisé en ce que** la structure de support (1) a été insérée dans un conteneur (14) qui est soumis une pression de gaz ou de liquide qui varie au moyen d'un milieu sous pression (19).

15. Procédé selon la revendication 13 ou 14, **caractérisé en ce qu'**un film protecteur (20) a été placé autour de la structure de support (1) et il forme un espace sous pression autour de celle-ci et il est soumis à des charges de pression sur le côté dirigé à l'opposé par rapport à la structure de support (1).

16. Procédé selon la revendication 1, **caractérisé en ce que** la couche de frontière (4) est appliquée à l'extérieur de la structure de support (1) après que la structure de support a été munie des cellules et du milieu de nutriment.

17. Procédé selon la revendication 1, **caractérisé en ce que** la structure de support (1) a été munie d'entrées et de sorties d'alimentation et de décharge (5, 6) pour les nutriments.

18. Procédé selon la revendication 17, **caractérisé en ce que** la structure de support (1) a été insérée dans un circuit de nutriment (11).

19. Utilisation d'une structure de support microporeuse ou grossièrement poreuse comme bioréacteur pour la production d'implants de dimension définissable, dans laquelle la structure de support (1) a été formée en tant que matériau d'accueil de colonisation biodégradable enlevable ou convertible pour des cellules (2) qui sont introduites dans la structure de support, et a la forme et la dimension de l'implant qui doit être formé à partir des cellules (2) employées et a sur l'extérieur une couche de frontière (4) qui est imperméable aux cellules et qui est enlevée après achèvement du processus de mise en culture des cellules.
